# EUROPEAN PATENT SPECIFICATION

(11) **EP 2 893 036 B1**
(45) Date of publication and mention of the grant of the patent: **11.01.2017**
(21) Application number: 13735256.3
(22) Date of filing: 08.07.2013
(51) Int. Cl.: C12Q 1/68

(54) **ASSOCIATION OF VASCULAR ENDOTHELIAL GROWTH FACTOR GENETIC VARIANT WITH METABOLIC SYNDROME**
ASSOZIATION GENETISCHER GEFÄSSENDOTHEL-WACHSTUMSFAKTORVARIANTEN MIT DEM STOFFWECHSELSYNDROM
ASSOCIATION DE VARIANT GÉNÉTIQUE DU FACTEUR DE CROISSANCE DE L'ENDOTHÉLIUM VASCULAIRE AVEC SYNDROME MÉTABOLIQUE

(30) Priority: 06.07.2012 GB 201212094
(43) Date of publication of application: 15.07.2015
(73) Proprietor: Randox Laboratories Ltd., Crumlin, County Antrim BT29 4QY (GB)
(72) Inventor: VISVIKIS-SIEST, Sophie, 54000 Nancy (FR)
(86) International application number: PCT/EP2013/064417
(87) International publication number: WO 2014/006231

(56) References cited:
- WO-A2-01/04351
- WO-A2-2012/024189
- A. T. KRAJA ET AL: "A Bivariate Genome-Wide Approach to Metabolic Syndrome: STAMPEED Consortium", DIABETES, vol. 60, no. 4, 8 March 2011 (2011-03-08), pages 1329-1339, XP55074098, ISSN: 0012-1797, DOI: 10.2337/db10-1011 cited in the application
- S. DEBETTE ET AL: "Identification of cis- and trans-Acting Genetic Variants Explaining Up to Half the Variation in Circulating Vascular Endothelial Growth Factor Levels", CIRCULATION RESEARCH, vol. 109, no. 5, 14 July 2011 (2011-07-14) , pages 554-563, XP55074095, ISSN: 0009-7330, DOI: 10.1161/CIRCRESAHA.111.243790 cited in the application

## Description

### BACKGROUND OF THE INVENTION

Metabolic syndrome (MetS) refers to a cluster of metabolic risk factors linked with impaired angiogenesis and often insulin resistance. In its advanced form, clinical fasting hyperglycemia or even type 2 diabetes is present. The syndrome has been broadened to encompass features such as central obesity, glucose abnormalities, dyslipidemia, elevated blood pressure, and low grade inflammation state with a pro-thrombotic circumstance ('components' of MetS). The risk of atherosclerotic cardiovascular disease and type 2 diabetes is greatly increased in patients with MetS. The prevalence of MetS in adults in developed countries, mainly due to the emerging "obesity epidemic", is as high as 35-40%. Several studies have revealed that ischemic tissues neovascularization is impaired in type 2 diabetes with obesity, hypercholesterolemia, or hypertension. Vascular endothelial growth factor (VEGF) is a multifunctional cytokine and is functional in angiogenesis, lymphangiogenesis, vascular permeability, and hematopoiesis. Adipocytes produce VEGF, which may act as an angiogenic and vascular survival factor for the omental vasculature. Elevated circulating levels of VEGF have been observed in ischemic heart disease, heart failure and stroke and in various other disorders such as type 2 diabetes and polycystic ovary disease. A positive association of VEGF circulating levels with MetS as well as with a number of its components has been shown (Lieb *et al.* 2009). Four single nucleotide polymorphisms (SNPs), explaining ∼50% of VEGF heritability, were recently identified using a genome-wide association study (GWAS) (Debette *et al.* 2011). Furthermore, a significant number of genetic variants have been shown to be associated with MetS and its components (Kraja *et al.* 2011; Kristiansson *et al.* 2012). WO2012024189 A2 describes a SNP in the SIRT-3 gene as a marker for metabolic syndrome.

There are numerous criteria used by various international bodies to classify MetS (e.g. Grundy *et al.* 2004 and 2005) including abdominal obesity (usually waist circumference, various cut-offs), BMI (various cut-offs), systolic blood pressure (various cut-offs), diastolic blood pressure (various cut-offs), antihypertensive medication, type 2 diabetes, family history of type 2 diabetes/CVD/hypertension, impaired fasting glucose, impaired glucose tolerance, 2-hour post glucose challenge, triglyceride levels and HDL cholesterol levels (various cut-offs). This lack of an internationally agreed criteria has purportedly contributed to the varied prevalence of MetS that has been reported in various studies (Cai *et al.* 2012). Thus MetS diagnosis is based on varied criteria each involving several individual test measurements; a more simplistic and definitive testing method for MetS diagnosis is, therefore, desirable.

### FIGURES

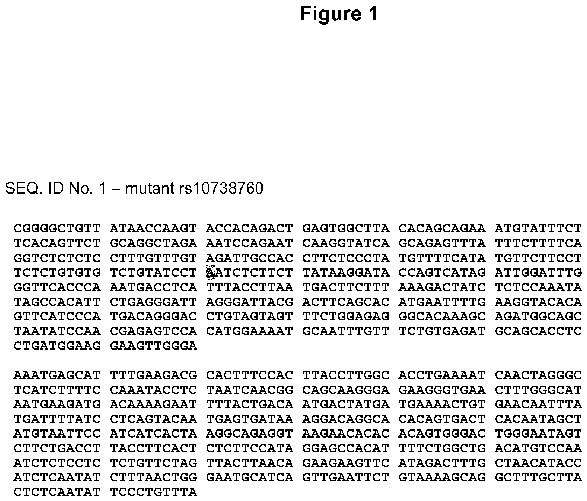

Figure 1 nucleotide sequence of the wild-type mutant rs10738760

### References

Cai H. et al. (2012). Prevalence and Determinants of Metabolic Syndrome among Women in Chinese Rural Areas. Plos One, 7(5): e36936.
Debette S. et al. (2011). Identification of cis- and trans-acting genetic variants explaining up to half the variation in circulating vascular endothelial growth factor levels. Circ. Res., 109: 554-563.
Kraja A.T. et al. (2011). A bivariate genome-wide approach to metabolic syndrome: STAMPEED consortium. Diabetes, 60: 1329-1339.
Kristiansson K. et al. (2012). Genome-Wide Screen for Metabolic Syndrome Susceptibility Loci Reveals Strong Lipid Gene Contribution but No Evidence for Common Genetic Basis for Clustering of Metabolic Syndrome Traits. Circ. Cardiovasc. Genet., 5: 242-249.
Grundy S.M. et al. (2004). Definition of Metabolic Syndrome: Report of the National Heart, Lung, and Blood Institute/American Heart Association Conference on Scientific Issues Related to Definition. Circulation, 109: 433-438.
Grundy S.M. et al. (2005). Diagnosis and Management of the Metabolic Syndrome: An American Heart Association/National Heart, Lung, and Blood Institute Scientific Statement. Circulation, 112: 2735-2752.
Lieb W. et al. (2009). Vascular endothelial growth factor, its soluble receptor, and hepatocyte growth factor: clinical and genetic correlates and association with vascular function. Eur. Heart J., 30: 1121-1127.
Zabaneh D. and Balding D.J. (2010). A genome-wide association study of the metabolic syndrome in Indian Asian men. PLoS One, 5: e11961.
Herbeth B. et al. (2010). Metabolic syndrome-related composite factors over 5 years in the STANISLAS family study: genetic heritability and common environmental influences. Clin. Chim. Acta, 411: 833-9.
Azimi-Nezhad M. et al. (2012). High Prevalence of Metabolic Syndrome in Iran in Comparison with France: What Are the Components That Explain This? Metab. Syndr. Relat. Disord.*,*
Nezhad M.A. et al. (2008). Metabolic syndrome: its prevalence and relationship to socio-economic parameters in an Iranian population. Nutr. Metab. Cardiovasc. Dis., 18(3): e11-e12.
Ehrich M. et al. (2005). Multiplexed discovery of sequence polymorphisms using base-specific cleavage and MALDI-TOF MS. Nucleic Acid Res., 33(4): e38.
Hindorff L.A. et al. (2009). Potential etiologic and functional implications of genome-wide association loci for human diseases and traits. Proc. Natl. Acad. Sci., 106: 9362-9367.
Yu W. et al. (2008). A navigator for human genome epidemiology. Nat. Genet., 40: 124-125.
Purcell S. et al. (2007). PLINK: a tool set for whole-genome association and population-based linkage analyses. Am. J. Hum. Genet., 81: 559-575.

### SUMMARY OF THE INVENTION

The pathological outcomes of metabolic syndrome are type 2 diabetes and cardiovascular disease. Currently, its diagnosis is dependent upon non-standardized criteria and requires physical examination and several biochemical assays. Following the identification of four SNPs which explained the heritibality of serum VEGF levels (rs6993770, rs6921438, rs4416670 and rs10738760 - Debette *et al.* 2011), the association of MetS and its components with genetic variants, and the possibility of a relationship between VEGF and MetS, it was decided to test whether one or more of the four previously discovered SNPs was associated with MetS and its components. The invention describes the identification of a single nucleotide polymorphism, rs10738760, whose absence is predictive of increased risk of metabolic syndrome. The identification of this genetic component to metabolic syndrome aids the implementation of standardized criteria for recognizing the condition and enables a simple, cost-effective method and kit to help predict the presence or absence of MetS.

### DETAILED DESCRIPTION OF THE INVENTION

The present disclosure describes a method of aiding the diagnosis of metabolic syndrome in a patient comprising detecting the presence or absence of the single nucleotide polymorphism rs10738760 in an *in vitro* sample taken from the patient. rs10738760, first identified in a GWAS VEGF heritability study, is located on chromosome 9p24.2 and is identified herein as base 201 of SEQ. ID No. 1. The mutant nucleotide at this position is adenine (A) and its occurrence in an *in vitro* patient sample is not associated with an increased or decreased likelihood that the patient has MetS; the presence of guanine (G), the wild type nucleotide, in an *in vitro* patient sample, is associated with an increased likelihood that the patient has MetS. The sample is preferably a nucleic acid sample, the original patient sample, as is standard in the art, having undergone any necessary preparatory steps prior to analysis.

Also described are kits for detecting mutant and/or wild type rs10738760 which include either a single probe to wild type or mutant rs10738760 or two probes, one probe specific to mutant rs10738760 and one probe specific to wild type rs10738760. The kits are preferably used to help diagnose metabolic syndrome.

As used herein, rs10738760 refers to the mutant rs10738760 (nucleotide at base 201 SEQ. ID No. 1= adenine), unless otherwise qualified; for example, reference to wild type rs10738760 implies the nucleotide guanine (G) is present at base 201 of SEQ. ID No. 1. As used herein, an 'allelic variation' refers to a variation in the nucleic acid and typically primary amino acid sequence of a gene in one or more alleles in a subject, such as a human patient. Allelic variations include single or multiple nucleic acid and amino acid substitutions, additons or deletions that have any one of a number of effects on protein expression, including: promoter activity that regulates transcription, frame-shrift, early protein termination, protein mis-folding, altered protein processing, destruction (or enhancement) of active sites or binding sites of a protein, mis-splicing of a mRNA or any other property of a nucleic acid or protein that effects the expression and/or function of the final gene products.

The source of the sequence referenced herein is the dbSNP database of the NCBI (http://www.ncbi.nim.nih.gov/snp). Detailed methods used to detect the rs10738760 SNP are described in *Debette et al.* (2011) and the Supplementary Material (Data Supplement at http://circres.ahajournals.org/content/ 109/5/554/suppl/DC1). A brief overview of these methods follows; reference to example(s) are not to be construed as being limited to only the described example(s).

A large number of methods, including high throughput methods, are available for detection of SNPs and /or other allelic variations, for example the PCR and restriction fragment length polymorphisms methods. DNA from a sample is sequenced (re-sequenced) by any method to identify a SNP or small allelic variation. A large variety of re-sequencing methods are known in the art, including high-throughput methods. Amplification-based methods are also available to identify allelic variations, such as SNPs, including: PCR, reverse transcriptase PCR (RT-PCR), isothermic amplification, nucleic acid sequence based amplification (NASBA), 5' fluorescence nuclease assay (for example TAQMAN assay), molecular beacon assay and rolling circle amplification. Other methods such as Restriction Fragment Length Polymorphisms RFLP, also may be employed - as is appropriate to identify variant allele(s). Assays may be multiplexed, meaning two or more reactions are conducted simultaneously in the same physical location such as in the same tube or on the same substrate such as a biochip, ensuring the reaction products of the multiplexed reactions can be distinguished. For example, TAQMAN or molecular beacon assays can be multiplexed by use of and by monitoring of accumulation or depletion of two different flurochromes corresponding to two different sequence-specific probes. In most cases, the appropriate method depends upon personal experience, available equipment and reagents, the need for high throughput and/or multiplexed methods, cost, required accuracy, and the skill levels of the technicians responsible for assay implementation. Design and implementation of these techniques are broadly-known and readily applicable by the skilled person in the art. In the implementation of the methods described herein, an array may be utilized. Arrays are particularly useful for high-throughput assays. The array typically comprises one or more reagents, for example, nucleic acid primers and/or probes, for identifying in a nucleic acid sample from a subject the occurrence of an allelic variation corresponding to one or more SNPs. As used herein, the term 'array' refers to reagents facilitating identification of allelic variations ina gene located at two or more identifiable locations. In one embodiment, an array is an apparatus at two or more discrete, identifiable reaction chambers, such as a 96 well dish, in which reactions comprising identified constituents are performed. In an exemplary embodiment, two or more nucleic acid primers or probes are immobilized onto a substrate in a spatially addressablemanner so that each individual primer or probe is located at a different and (addressable) identifiable location on the substrate. Substrates include multi-well plates, ceramic chips and beads. In one embodiment, the array comprises two or more sets of beads, with each bead having an identifiable marker, such as a quantum dot or fluorescent tag, so that the beads are individually identifiable using, for example, a flow cytometer. In one embodiment, in the context of the present disclosre, an array may be a multi-well plate containing two or more well reaction chambers with primers for amplifying DNA to identify SNPs or probes for binding specific sequences. As such, reagents, such as probes and primers may be bound or otherwise deposited onto or into specific locations on an array. Reagents may be in any suitable form, including: in solution, dried, lyophilized or glassified. Useful array technologies include, for example, Affymetrix GeneChip® Array, GenChip® CustomSeq® Resequencing Arrays (commercially available from Affymetrix Inc. of Santa Clara, California). Informatics and/or statistical software or other computer-implemented processes for analyzing array data and/or identifying genetic risk factors from data obtained from a patient sample, are known in the art.

### Materials and Methods

### Study population

This study is based on the STANISLAS Family study (SFS), a 10-year longitudinal survey involving 1,006 volunteer families from Vandoeuvre-lès-Nancy, France (Herbeth *et al.* 2010). Individuals with known acute or chronic diseases such as stroke, myocardial infarctions, hypertension, dyslipidemia or cancer were not included, as the aim of the study was the assessment of genetic susceptibility factors on the variability of intermediate phenotypes in physiological conditions without the influence of any long term medication and disease. The study protocol was approved by the Local Ethics Committee of Nancy and all subjects gave written informed consent for their participation in the study. A total of 403 unrelated adults were involved in the current study. All data were collected during the second examination of the SFS.

### Anthropometric indices, medical history and lifestyle data

Weight and height were measured in standing position with light clothing without shoes. Weight was recorded with digital scales to the nearest 200 grams using a weight scale. Height was measured to the nearest 0.1 cm using wall-mounted stadiometer, with the subjects' shoulders in a normal position. Body mass index (BMI) was calculated according to the Quetelet's formula: weight (kg)/height (m²). Waist circumference was taken at the midpoint between the lower margin of the last palpable rib and the top of the iliac crest (hip bone), and hip circumference was measured at the maximum level over light clothing, using a standard tape measure, without any pressure on the body surface. Measurements were recorded to the nearest 0.1 cm. All measurements were taken by trained nurses according to standard procedures, and the reliability of the measuring devices was periodically checked during the study period. SBP and DBP were calculated as the mean of three measurements taken under standardized conditions with a sphygmomanometer, with the subject in a supine position (Azimi-Nezhad *et al.* 2012). Pulse pressure was calculated as the difference between SBP and DBP. Also, data were collected using standard questionnaire including information about lifestyle such as smoking and personal medical history. Hypertension was defined as SBP >140mmHg and/or DBP >90mmHg. Central obesity was defined as a WC >102 cm in men and >9 cm in women, while general obesity was defined as BMI ≥30Kg/m² (Azimi-Nezhad *et al*. 2012). MetS was defined based on the International Diabetes Federation (IDF) criteria (Nezhad *et al.* 2008): WC ≥94 cm in men or ≥80 cm in women plus any two of the four following criteria i) triglyceride ≥1.7 mmol/l or drug treatment for elevated triglyceride, ii) high-density lipoprotein cholesterol (HDL-C) < 1.03 mmol/l in men or <1.3 in women or having medication for reduced HDL-C, iii) SBP ≥ 130 mmHg or DBP ≥ 85 mmHg or anti-hypertensive medication, iv) fasting blood glucose ≥ 5.6 mmol/l or drug treatment for increased serum glucose.

### Laboratory measurements

Blood samples were collected after overnight fast between 8:00 and 9:00 am. or 11:00 and 12:00 a.m. Serum and plasma samples were separated by centrifugation at 2000×g for 15 min. Serum fasting blood glucose, total cholesterol and triglycerides levels were measured using standard enzymatic methods (Merck, Germany) on an automated analyzer AU5021 (Olympus, Japan). Insulin, apolipoprotein A-I, apolipoprotein B, apolipoprotein CIII were determined by immunonephelometry on Behring Nephelometer analyzed with Behring reagents (France). Apolipoprotein E and HDL-C were measured by turbidimetry and precipitation by phosphotungstate respectively, on a Cobas-Mira analyzer (Roche). VEGF plasma levels quantification was performed by Randox Ltd (Crumlin, UK) using a biochip array analyser (Evidence ®) (Herbeth et al. 2010).

### Genotyping

The SNPs rs6921438, rs4416670, rs6993770, rs10738760 were genotyped by Genoscreen (http://genoscreen.fr), using a Sequenom iPLEX Gold assay-Medium Throughput Genotyping Technology (Ehrich et al. 2005).

### Statistical analysis

Continuous variables are presented as mean value ± standard deviation and categorical variables are given in percentages. Hardy-Weinberg equilibrium was tested using the chisquare test. All continuous variables were log-transformed to normalize their distribution. For the SNPs associations analyses an additive model was used. Results are presented using the minor allele as reference allele. Logistic regression models adjusted for age, gender and BMI were used to test possible associations between VEGF-related SNPs and VEGF plasma levels with MetS. Subsequent analyses adjusted for age, gender and smoking were performed. Similar models adjusted for age and gender were used for general obesity and central obesity and models adjusted for age, gender and BMI were applied for the associations with hypertension. Concerning the quantitative traits including waist circumference, waist-to-hip ratio, waist-to-height ratio, BMI, SBP, DBP, fasting blood glucose, insulin and lipids linear regression models adjusted for age, gender and BMI were applied for the assessment of possible effects of the VEGF-related SNPs (the model for the assessment of association with BMI was adjusted for age and gender only). All analyses were performed using PLINK 1.07 software (http://pngu.mgh.harvard.edu/purcell/plink) (Purcell *et al.* 2007) and the SPSS statistical software version 16.0 (SPSS, Inc, Chicago, Illinois). Significance was assessed at a two-tailed P=0.05 level. The significant results of this study were compared to previous findings in the literature (previous GWAS), by imputation analyses using Plink. SNPs with a correlation coefficient ≥ 80% were considered in linkage disequilibrium (LD). The GWAS investigator of HuGENavigator engine (Yu *et al.* 2008) and the NHGRI Catalog of published GWAS (http://www.genome.gov/gwasstudies) (Hindorff *et al.* 2009) were used in order to assess previous GWAS concerning blood lipid levels.

### Results

The general characteristics of the studied population and polymorphisms are presented in Tables 1 and 2 respectively. No statistically significant associations were observed between VEGF plasma levels and MetS as well as with its related components in this population. Regarding the effect of the assessed SNPs, rs10738760 was significantly associated with MetS (Table 3). The presence of the minor allele A was associated with decreased risk for MetS. The result remained significant after adjustment for smoking. We also observed a trend for association between rs10738760 and triglycerides levels (beta=-0.028, P-value=0.056). There was no association of this SNP with other lipid profile molecules.

The three previous GWAS (Kraja *et al.* 2011; Kristiansson *et al.* 2012; Zabaneh *et al.* 2010) conducted in relation to MetS and its components did not address the rs10738760 SNP (genotyped or in linkage disequilibrium with another SNP). Therefore, this is considered as a novel polymorphism for MetS.

**Table 1: Characteristics of STANISLAS Family Study population**

| **Variable** | | **Adults (n=403)** | |
|---|---|---|---|
| | | **Mean** | **SD** |
| Age (years) | | 44.460 | 4.885 |
| Gender (%) | Male | 50.4 | |
| | Female | 49.6 | |
| Smoking (%) | No | 45 | |
| | Yes | 25.4 | |
| | Ex-smoker | 29.6 | |
| BMI (Kg/m²) | | 24.904 | 3.880 |
| Waist circumference (cm) | | 82.433 | 11.018 |
| Waist/Hip ratio | | 0.844 | 0.089 |
| Waist/Height ratio | | 48.962 | 5.850 |
| Systolic blood pressure (mmHg) | | 123.29 | 13.79 |
| Diastolic blood pressure (mmHg) | | 79.93 | 10.130 |
| Pulse pressure (mmHg) | | 49.35 | 8.75 |
| Fasting blood glucose (mmol/l) | | 5.033 | 0.682 |
| Insulin (IU/ml) | | 6.210 | 5.644 |
| Triglycerides (mmol/l) | | 1.343 | 1.960 |
| Low-density lipoprotein-C (mmol/l) | | 3.558 | 0.898 |
| High-density lipoprotein-C (mmol/l) | | 1.606 | 0.465 |
| Total cholesterol (mmol/l) | | 5.744 | 1.001 |
| Apolipoprotein A1 (g/l) | | 1.630 | 0.259 |
| Apolipoprotein B (g/l) | | 1.013 | 0.233 |
| Apolipoprotein E (mg/l) | | 41.816 | 17.574 |
| Apolipoprotein CIII (mg/l) | | 98.330 | 37.193 |
| Vascular endothelial growth factor (ng/l) | | 42.748 | 43.330 |

**Table 2: Genetic variants' characteristics of STANISLAS cohort study population**

| **Chromosome** | **SNP** | **Function** | **Closed to/on gene** | **Minor allele** | **Common allele** | **Minor allele frequency (MAF)** |
|---|---|---|---|---|---|---|
| 6 | rs6921438 | Intergenic | near of VEGF | A | G | 0.445 |
| 6 | rs4416670 | Intergenic | near of VEGF | C | T | 0.476 |
| 8 | rs6993770 | Intronic | between ZFPM2 and LRP12 genes | T | A | 0.283 |
| 9 | rs10738760 | Intergenic | between VLDLR and KCNV2 | A | G | 0.491 |

**Table 3: Associations of the 4 VEGF single nucleotide polymorphisms with metabolic syndrome in adults from the STANISLAS population (n=403) (model adjusted for age,gender and BMI)**

| | **RS6921438** | | **RS4416670** | | **RS6993770** | | **RS10738760** | |
|---|---|---|---|---|---|---|---|---|
| | OR (95% CI) | P-value | OR (95% CI) | P-value | OR (95% CI) | P-value | OR (95% CI) | P-value |
| **Metabolic syndrome** | 1.363 (0.78-2.37) | 0.272 | 0.7545 (0.43-1.31) | 0.320 | 0.7938 (0.43-1.45) | 0.454 | 0.4755 (0.27-0.84) | **0.010** |

| | | | | | | | | |
|---|---|---|---|---|---|---|---|---|
| OR: Odds ratio; CI: Confidence Interval | | | | | | | | |

## Claims

1. A method of aiding the diagnosis of metabolic syndrome in a patient comprising detecting the presence of wild type or mutant rs10738760 in an *in vitro* sample taken from the patient in which the presence of wild type rs10738760 (G allele) indicates that the patient has an increased likelihood of metabolic syndrome, whereas detection of mutant rs10738760 (A allele) indicates that the patient does not have an increased likelihood of metabolic syndrome.

2. Use of a kit in the diagnosis of metabolic syndrome which includes either one or two nucleic acid probes; the single probe kit includes a nucleic acid probe which specifically binds to either mutant rs10738760 (A allele) or wild type rs10738760 (G allele), the two probe kit includes a nucleic acid probe which specifically binds to wild type rs10738760 (G allele) and a nucleic acid probe which specifically binds to mutant rs10738760 (A allele).

## Patentansprüche

1. Verfahren zum Unterstützen der Diagnose von metabolischem Syndrom in einem Patienten, umfassend das Nachweisen des Vorliegens von Wildtyp- oder mutiertem rs10738760 in einer *in-vitro*-Probe, genommen von dem Patienten, wobei das Vorliegen von Wildtyp-rs10738760 (G-Allel) andeutet, dass für den Patienten eine erhöhte Wahrscheinlichkeit für metabolisches Syndrom vorliegt, wobei der Nachweis von mutiertem rs10738760 (A-Allel) andeutet, dass für den Patienten keine erhöhte Wahrscheinlichkeit für metabolisches Syndrom vorliegt.

2. Gebrauch eines Kits beim Diagnostizieren von metabolischem Syndrom, das wenigstens eine oder zwei Nukleinsäuresonden enthält; wobei das Einzelsondenkit eine Nukleinsäuresonde enthält, die spezifisch entweder an mutiertes rs10738760 (A-Allel) oder Wildtyp-rs10738760 (G-Allel) bindet, wobei das Zwei-Sonden-Kit eine Nukleinsäuresonde, die spezifisch an Wildtyp-rs10738760 (G-Allel) bindet, und eine Nukleinsäuresonde, die spezifisch an mutiertes rs10738760 (A-Allel) bindet, enthält.

## Revendications

1. Procédé d'aide au diagnostic du syndrome métabolique chez un patient comprenant la détection de la présence de rs10738760 de type sauvage ou mutant dans un échantillon *in vitro* prélevé sur le patient, dans lequel la présence de rs10738760 de type sauvage (allèle G) indique que le patient a plus de risques de souffrir du syndrome métabolique, tandis que la détection de rs10738760 mutant (allèle A) indique que le patient n'a pas plus de risques de souffrir du syndrome métabolique.

2. Utilisation d'un kit lors du diagnostic du syndrome métabolique qui inclut soit une soit deux sondes d'acide nucléique ; le kit à une sonde comprend une sonde d'acide nucléique qui se lie spécifiquement soit à rs10738760 mutant (allèle A) soit à rs10738760 de type sauvage (allèle G), le kit à deux sondes comprend une sonde d'acide nucléique qui se lie spécifiquement à rs10738760 de type sauvage (allèle G) et une sonde d'acide nucléique qui se lie spécifiquement à rs10738760 mutant (allèle A).
